# EUROPEAN PATENT SPECIFICATION

(11) **EP 1 032 402 B1**
(45) Date of publication and mention of the grant of the patent: **25.02.2004**
(21) Application number: 98960416.0
(22) Date of filing: 24.11.1998
(51) Int. Cl.: A61K 31/66, A61P 43/00

(54) **Use of citicoline for the treatment of multiple sclerosis (MS)**
Verwendung von CITICOLINE ZUR BEHANDLUNG VON MULTIPLER SKLEROSE
Utilisation de la CITICOLINE pour le traitement de la SCLEROSE EN PLAQUES

(30) Priority: 26.11.1997 US 66556 P; 01.12.1997 US 67017 P; 06.08.1998 US 129778
(43) Date of publication of application: 06.09.2000
(73) Proprietor: INTERNEURON PHARMACEUTICALS INCORPORATED, Lexington, MA 02173 (US)
(72) Inventor: SANDAGE, Bobby W. Jr., Lexington, MA 02421 (US); COOPER, Glenn, L., Sudbury, MA 01776 (US); LOCKE, Kenneth, W., Littleton, MA 01460 (US); KIYOIZUMI, Takashi, Wellesley Hills, MA 02481 (US)
(74) Representative: Luderschmidt, Schüler & Partner GbR
(86) International application number: PCT/US1998/025051
(87) International publication number: WO 1999/026633

(56) References cited:
- EP-A- 0 147 185
- CN-A- 1 138 985
- DE-A- 3 400 276
- MAPELLI G.: "clinical observations on a group of patients with multiple sclerosis treated with citicoline" ARCISPEDALE S. ANNA DI FERRARA, vol. 28, no. 3-4, 1975, pages 267-273, XP002094876
- BERKOW ET AL: "The Merck Manual of diagnosis and therapy" 1982 , MERCK SHARP AND DOHME RES. LAB. , RAHWAY N. J. XP002094878 see page 1356: "treatment"
- AGUT ET AL: "oral CDP-choline administration to rats increases brain phospholipid levels" ANN. N.Y. ACAD. SCI., vol. 695, 24 September 1993, pages 318-320, XP002094877

## Description

### Field Of The Invention

The present invention relates to a method of treating motor neuron and demyelinating diseases, including multiple sclerosis (MS). In particular, the invention relates to the use of citicoline (cytidine-5'-diphosphocholine or CDP-choline) in the treatment of these diseases.

### Background Of The Invention

There are a large number of identifiable motor neuron diseases and demyelinating diseases. Among the most common diseases of these types is MS.

Of the various demyelinating diseases, MS is by far the most well known. The causative agent of MS is unknown, although both infectious agents and autoimmunity are suspected. MS often strikes in early adulthood and is characterized by the formation of lesions (demyelinated plaques) in the central nervous system. The afflicted individual can exhibit lack of coordination, dysarthria (slurred speech), numbness, paralysis and/or urinary incontinence. Blindness has also been reported. The course of MS often involves spontaneous remission followed by relapses. Fortunately, in a majority of cases, permanent remission eventually occurs, but only after successively less severe relapses, and with an average duration of illness spanning 27 years.

Despite the fact that many MS patients experience eventual remission, the long life span of the disease inflicts psychological and economic hardship on patient and caregiver alike. Hence, treatment of at least the symptoms of the disease is highly advantageous.

Although there are some treatments for these diseases, there remains a need in the art for a method of treating motor neuron and demyelinating diseases, such as MS, which provides for both increased relief of symptoms and at least temporary cessation or even reversal of neuronal damage.

### Summary Of The Invention

The present invention concern the use of citicoline or a pharmaceutically-acceptable salt thereof for the preparation of a medicament for the treatment of MS. In another embodiment, an effective amount of a combination of citicoline and a glucocorticoid, preferably dexamethasone, prednisone or methylprednisolone is to be administered. The present invention also relates to the use of citicoline for the preparation of a pharmaceutical medicament for the treating of MS, comprising admixing an effective amount of citicoline with a pharmaceutically acceptable carrier.

It is, therefore, one object of the invention to improve the treatment of symptoms of those afflicted with MS.

Another object of the invention is to decrease symptoms in patients who have suffered nerve injury or nerve death due to MS.

Still another object of the invention is to prevent the worsening of symptoms over the course of the disease, i.e., to inhibit progression.

These and other objects of the invention will be apparent to those of ordinary skill in view of the discussion above and the additional detailed description provided below relating to preferred embodiments of the invention.

### Detailed Description Of The Preferred Embodiments

Citicoline is believed to have multiple therapeutic effects. For example EP 0 147 185 A2 (MIT) discloses the use of citicoline for the treatment of diseases which affect acetyl-choline containing neurons in the brain and other tissues and Mapelli, G. (1975, Arcispedale S. Anna di Ferrara 20, 267-73) discloses the use of citicoline for the treatment of amyotrophic lateral sclerosis (ALS). Although the relative contribution of each effect on the treatment of motor neuron or demyelinating disease is unknown, citicoline and its metabolites -- which include cytidine and choline -- are believed to play important roles in the generation of phospholipids involved in membrane formation and repair. These compounds also are believed to contribute to critical metabolic functions, such as the formation of nucleic acids, and the synthesis of the neurotransmitter acetylcholine. Thus, under conditions of frank neuronal damage with associated nerve cell degeneration, citicoline may function to: (1) stabilize membranes by providing substrates for membrane maintenance; (2) repair damaged membranes by supplying important substrates for membrane formation; and, (3) restore neuronal function by supplying a substrate for the formation of acetylcholine. Moreover, unlike other proposed therapeutic agents, citicoline has the potential not only to stabilize the size or locale of the area of damage, but also to contribute to the repair of the damaged area.

Without being limited by theory, it is believed that citicoline has at least a dual mechanism of action: limiting nerve damage and further progression of disease and aiding in the repair of damaged neuronal tissues. Administration of citicoline is believed to limit the extent of the tissue damage by preventing the accumulation of toxic free fatty acids. In addition, following its administration, it is believed that citicoline is broken down into components, including cytidine and choline, which are substrates required in the formation of phosphatidylcholine, the primary phospholipid of nerve cell membranes, via the Kennedy pathway. It is further postulated that to normalize brain and/or muscle function, nerve cells damaged by motor neuron or demyelinating diseases must manufacture new membrane elements. As described below, in preclinical animal models of ALS and MS, administration of citicoline is shown to reduce the functional deficits produced by nerve degeneration.

Citicoline is preferably administered orally as a pharmaceutically acceptable salt. The preferred salt is the monosodium salt of citicoline, as this form is readily available in pharmaceutically acceptable purity. Citicoline monosodium is an exogenous form of cytidine-5'-diphosphocholine (CDP-choline). Endogenous CDP-choline is a key intermediate in the biosynthesis of membrane phosphatidylcholine, the primary lipid membrane component involved in the dynamic regulation of cellular integrity.

Citicoline may be administered in the following daily dosages. All dosages are provided on a citicoline monosodium basis and on a per patient basis (ranging from about 45 kg to about 100 kg per patient or 70 kg patient on average).

Generally daily citicoline dosages may range from about 100 mg to about 5000 mg, desirably from about 250 to about 3000 mg and preferably from about 500 to about 2000 mg. Doses may be administered once or up to four or more times daily. A highly preferred dosage is 500 mg administered twice per day per patient. If greater therapeutic efficacy is required, a preferred administration is 2000 mg administered in either a single 2000 mg dose or two 1000 mg doses.

The treatment length is variable, but it has been observed that patients tolerate citicoline well at doses ranging from about 250 mg to about 2000 mg for prolonged periods, that is, from several weeks to several years. Dosages may be varied over time depending on the severity of symptoms, individual patient tolerance, route of administration and response to treatment. Treatment may be continued indefinitely if needed and if tolerated well.

Preferably, citicoline is administered orally in the form of capsules, cachets, tablets or lozenges, or as a powder or granules for reconstitution as a solution or suspension in an aqueous or non-aqueous liquid. Administration may also be in the form of a bolus, electuary, suppository, or paste. Formulations for inhalation, or intranasal administration are also contemplated.

Formulations of the active ingredient, suitable for parenteral administration, may comprise a sterile, aqueous preparation of the citicoline active ingredient. The formulations may be presented in unit dosage form and may be prepared by any of the methods well-known in the art of pharmacology.

In addition to containing the standard and well known pharmaceutical carriers and/or excipients, all of the above formulations may contain other therapeutically active substances. Thus, the present invention also contemplates a combination treatment regimen that relates to the co-administration of citicoline and at least a second therapeutic agent, or the respective pharmaceutically acceptable salts thereof.

Broad categories of the at least second therapeutic agent are contemplated. These agents include, but are not limited to, glutamate and glycine antagonists such as neurontin, and drugs such as ACTH, glucocorticoids (e.g., methylprednisolone, prednisone and dexamethasone), antiinflammatory drugs, diphenylhydramine, quinine, myotrophin, or IGF-1, BDNF, BFGF, beta-interferon, Betaseron, Copaxone, Baclofen, Riluzole, and epitopes of myelin basic proteins and the like, which are often used to treat ALS or MS.

Yet other therapeutic agents useful in combination with citicoline are calcium channel blockers (e.g., AJ-394, AK-275, Calpain inhibitors, CD-349, Clentiaze, CNS-1237, CNS-2103, CPC-304 and CPC-317, Dazodipine, Diperdinine, Emopamil, Fasudil, Lacidipine, Lifarizine, Lomerizine, Magnesium, MDL:28170, NB-818, Nilvadipine, Nimodipine, NS-626 and related compounds, SM-6586, SNX-111, S-312-d, U-92032, UK-74505, US-035 and the like), agents targeted at nitric oxide, agents targeted at various other neurotransmitters (e.g., alpha₂-receptor therapeutics, CV-5197, Dopamine receptors, Enadoline, Lazabemide, Milnacipran, Nalmefene, RP-60180, SR-57746A, synaptic uptake blockers and the like), cytokines, hormones and related products (e.g., AN-100225 and AN-100226, Calcitonin gene-related peptides, CEP-075 and related compounds, Ciliary neurotrophic factor, Endothelial cell factor, Endothelin inhibitors, FR-139317 Interleukin-1 receptor antagonist (lipocortin), JTP-2942, Macrophage-regulating compounds, Motoneuronetrophic factor NBI-117, Nerve growth factor, Neural stem cells, Neutrophil inhibitory factor, NS-506, NT-3, Posatirelin, Schwann cell promoters, sCR1, Somatomedin-1 and the like), free radical scavengers (e.g., EPC-K1, MCI-186, Nicaraven, Phenazoviridin, Resorstatin, Rumbrin, Superoxide dismutase, Tirilazad mesylate, U-88999E, Yissum project P-0619, YM-737 and the like), gangliosides and related products (e.g., LIGA4, LIGA4, Monosialoganglioside (GM1), ND-37, Siagoside and the like).

Still other classes of second therapeutic agents include, but are not limited to, modulators of various specific enzymes, neuroprotectives with "diverse" actions (e.g., Ademetionine sulphate tosilate, Ancrod, Apocuanzine, CPC-111, CPC-211, HSV vectors, KF-17329 and KF-19863, LY-178002, MS-153, Nicorandil, N-3393 and N-3398, SUN 4757, TJ-8007, VA-045 and the like, and imaging or contrast agents).

Therefore, the application concerns the use of citicoline for the preparation of a medicament for the treatment of MS, wherein the citicoline is to be co-administered with an effective amount of citicoline and at least a second therapeutic agent, or their respective pharmaceutically acceptable salts. The first dose may then be followed by the co-administration of one or more subsequent doses of effective amounts of citicoline alone, the at least second therapeutic agent alone, or their respective pharmaceutically acceptable salts, or as subsequent combinations thereof. Consistent with the other methods disclosed herein, the first dose may be co-administered after diagnosis. By the use of the term "co-administration," is meant that the citicoline and the at least second therapeutic agent, or their respective pharmaceutically acceptable salts, are administered together or sequentially.

The therapy includes the administration or co-administration of subsequent doses, which is preferably carried out over a period of at least about 30 days. In a specific embodiment of the invention, the co-administration of subsequent doses is carried out over a period of at least about 4-8 weeks, preferably over a period of at least about six months to about one year. Furthermore, the first dose or subsequent doses are co-administered one or more times daily over the predetermined period. It is anticipated that subjects who may benefit the most from the combination therapy are those who are in the acute, active stage of MS, or chronic progressive MS. Maintenance doses may be required for some patients for the rest of their lives.

In the composition, the effective amount of active ingredients in a therapeutic dose may vary according to the particular need. Typical ranges, however, may be from about 100 mg to about 5000 mg of citicoline and about 10 mg to about 1000 mg of at least a second therapeutic agent.

The present invention is illustrated by the Examples that follow, it being understood, however, that the invention is not limited to the specific details of these Examples.

### Example 1

### Experimental Allergic Encephalomyelitis (EAE) Model for MS

Experimental allergic encephalomyelitis (EAE) is an inflammatory autoimmune demyelinating disease which can be induced in laboratory animals by injection of myelin basic protein (MBP) or ground spinal cord from another species. This artificially induced disease has become the standard laboratory model for studying clinical and experimental autoimmune diseases. There are many similarities between EAE in animals and MS in humans, including chronic relapse. Thus, EAE is a good predictor of efficacy of drugs and drug combinations for treatment of various autoimmune diseases. Also, because of the similarity in motor symptoms, EAE may also be predictive of drug efficacy for ALS.

The EAE test model is employed to establish the activity of citicoline against MS. Such testing is conducted according to the following procedure.

Thirty female Lewis rats are injected in their foot pads with guinea-pig spinal cord homogenate in complete Freund's adjuvant. The rats are divided into three groups of 10 each. One group is administered citicoline i.p. daily in a dose of 500 mg/kg beginning at 9 days after inoculation. A second group is administered dexamethasone in daily doses of approximately 0.0375 mg/kg s.c., beginning 9 days after inoculation. The third group is a control to which is administered 0.9% saline solution beginning 9 days after inoculation. Duration of the treatment is approximately 17 days. Animals are examined daily, which consists of weighing and scoring for symptoms of EAE according to a disability scale of 0-4.

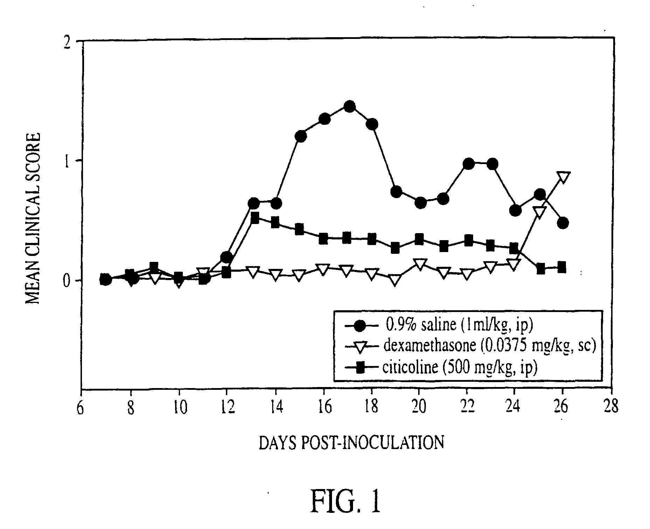

The results of such rat studies are shown in Figure 1. They establish that citicoline inhibits the progress of EAE, with a dose of 500 mg/kg exhibiting desirable levels of activity. Dexamethasone at a relatively high dosage also inhibits the progress of EAE initially.

### Example 2

This example demonstrates the benefits of prevention therapy with citicoline. Thirty-two female Lewis rats are divided into four groups of eight each and on day one administered saline, citicoline (500 or 1000 mg/kg, i.p.), or dexamethasone (0.0375 mg/kg), respectively. Dexamethasone acts as a positive control through its ability to suppress immune function. On day two, experimental autoimmune encephalomyelitis is induced in all rats by injecting ground guinea pig spinal cord in complete Freund's adjuvant in the foot pad. Therapy is continued daily. The rats are tested daily and assigned a functional score of 0 to 4, with 0 being normal and 4 representing death or inability to move. The results, which are summarized in the Table, indicate that administration of citicoline markedly reduces the rapid deterioration of motor function in the test subjects compared to the saline group. Numerical values are averages of the functional scores in each treatment group from Days 14-21 of the study.

**Table**

| Group | Mean Functional Score | | | | | | | |
|---|---|---|---|---|---|---|---|---|
| | Day 14 | Day 15 | Day 16 | Day 17 | Day 18 | Day 19 | Day 20 | Day 21 |
| vehicle (saline) | 0.72 | 0.94 | 1.0 | 1.12 | 1.16 | 1.19 | 1.35 | 1.25 |
| Dexamethasone (0.0375 mg/kg) | 0.14 | 0.42 | 0.0 | 0.14 | 0.28 | 0.64 | 0.57 | 0.64 |
| citicoline (500 mg/kg) | 0.43 | 0.41 | 0.37 | 0.38 | 0.28 | 0.59 | 0.68 | 0.87 |
| citicoline (1000 mg/kg) | 0.56 | 0.78 | 0.84 | 0.69 | 0.62 | 0.91 | 0.75 | 0.84 |

### Example 3

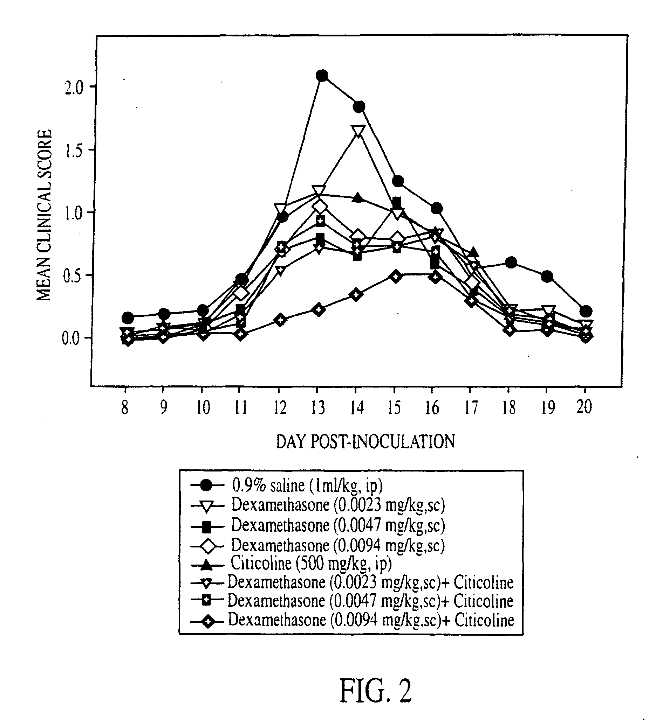

This example demonstrates the benefits of prevention therapy with a combination of citicoline and dexamethasone. Sixteen female Lewis rats are divided into eight groups of two each and on day one each group is administered one of the following eight solutions:
1. Dexamethasone (Dex) 9.375 µg/kg
2. Dexamethasone 4.6 µg/kg
3. Dexamethasone 2.3 µg/kg
4. Citicoline 500 mg/kg + Dex 9.375 µg/kg
5. Citicoline 500 mg/kg + Dex 4.6 µg/kg
6. Citicoline 500 mg/kg + Dex 2.3 µg/kg
7. Citicoline 500 mg/kg
8. Saline
On the same day, experimental autoimmune encephalomyelitis is induced in all rats by injecting ground guinea pig spinal cord in complete Freund's adjuvant in the foot pad. Therapy is continued daily. The rats are tested daily and assigned a functional score of 0 to 4, with 0 being normal and 4 representing death or inability to move. The results, which are summarized in Figure 2, indicate that administration of citicoline in combination with 2.3 µg/kg of dexamethasone reduces deterioration of motor function in the test subjects to a level equal to or better than that observed for administration of dexamethasone alone at levels up to 9.375 µg/kg. This enhancement of the activity of dexamethasone allows the same results to be obtained without higher doses of dexamethasone, which have the potential for toxic effects, as described in Goodman & Gilman's The Pharmacological Basis of Therapeutics, Ninth Edition (1996) at pages 1474-1476. In addition, administration of citicoline in combination with 9.375 µg/kg of dexamethasone reduces deterioration of motor function substantially compared to the other treatments illustrated in Figure 2. This increased activity is achieved without exposing the subject to the detrimental effects of higher steroid dosages.

### Example 4

This example demonstrates the benefits of post-symptom therapy with a combination of citicoline and dexamethasone. Eight female Lewis rats are divided into four groups of two each and on day one experimental autoimmune encephalomyelitis is induced in all rats by injecting ground guinea pig spinal cord in complete Freund's adjuvant in the foot pad. At symptom onset at approximately day 10, each group is administered one of the following four solutions:
1. Dexamethasone (Dex) 37.5 µg/kg
2. Citicoline 500 mg/kg + Dex 9.375 µg/kg
3. Citicoline 500 mg/kg
4. Saline, 0.9% (1 ml/kg, ip)
Therapy is continued daily. The rats are tested daily and assigned a functional score of 0 to 4, as follows:
0: normal
0.1: tail weaker than normal
0.25: tail fails to curl around examiner's finger
0.75: tail strength only at base
1.0: loss of all tail strength
1.5: limp tail + failure of one or more hind limbs to grip rotorod
1.5: hind limb dragging (weak movement possible)
1.75: hind limb dragging + failure of one or more hind limbs to grip rotorod
2.0: hind limb paralysis
3.0: hind limb paralysis + fail rotorod test
4.0: total paralysis or death

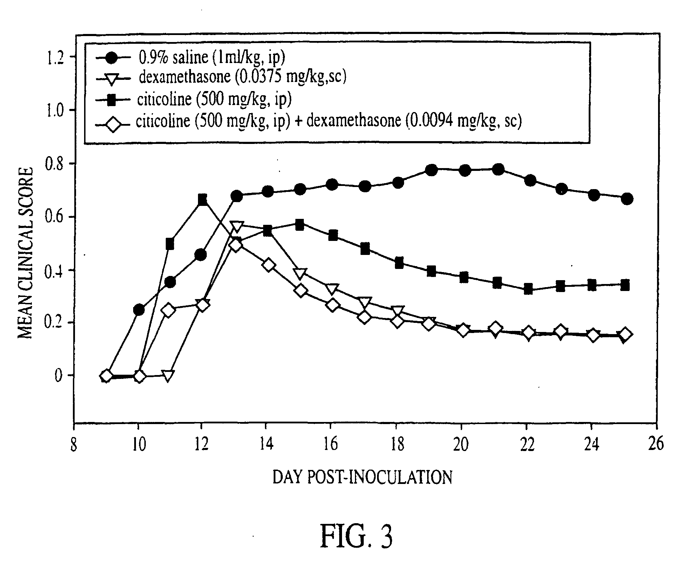

The results, which are summarized in Figure 3, indicate that administration of citicoline in combination with 9.375 µg/kg of dexamethasone reduces deterioration of motor function in the test subjects to a level equal to or better than that observed for administration of dexamethasone alone at a relatively high level of 37.5 µg/kg. This enhancement of the activity of dexamethasone allows the same results to be obtained without higher doses of dexamethasone in a post-symptom treatment regimen. In addition, administration of citicoline in combination with 9.375 µg/kg of dexamethasone reduces deterioration of motor function substantially compared to administration of citicoline alone. This increased activity is achieved without exposing the subject to the detrimental effects of higher steroid dosages.

### Examples 5-7 - Human Studies

### Example 5

A study of two sets of four patients each with chronic multiple sclerosis is undertaken. Each patient is first examined for normal hepatic, renal, and bone marrow functioning to establish baseline values. Each of the patients in each group is then treated either with citicoline dissolved in sterile preservative-free isotonic saline 10% or oral tablet or capsule. The citicoline is administered orally or intravenously at a dosage of 250, 500, or 1,000 mg each patient each day for six months. Patients are examined on a daily basis. During the treatment period, daily blood counts and twice weekly blood chemistries are performed on each patient. The neurologic function of each of these patients is measured using the expanded Krutzke disability status scale (EDSS), and the Scripps neurologic rating scale (SNRS).

There is no evidence of any significant toxic side effects. None of the eight patients exhibit any nausea, vomiting, skin rash, or hepatic or renal dysfunction.

In essence, there is no evidence of toxicity in these eight patients with normal marrow, hepatic and renal function. Likewise, the side effects of the citicoline are imperceptible in these eight patients.

Measurement of neurologic function using the EDSS and SNRS scales provides evidence of improvement in MS patients during treatment with citicoline.

### Example 6

Six patients ranging in age from 33 to 38 who have suffered from multiple sclerosis for more than 6 years and have been bedridden for at least 4 months are treated with steroids after which walking is possible with the aid of a walker. A wheelchair is needed for travel outside the home. The attending physician advises the patients that without chemotherapy (including treatment with cytoxan), the patients would return to the bedfast state within 6 months.

The patients elect to discontinue all prescribed therapy and begin taking approximately 1000 mg oral citicoline daily. The patients observe no change in the status of the disease until about 10 weeks have elapsed at which time they report feeling better than at any time in the previous year. Within two to four additional weeks, all patients are able to walk longer distances.

### Example 7

A.H. is a 54 year-old white, right-handed, married female who presents with chronic progressive multiple sclerosis. The patient reports first known neurological event to have occurred at 28 years old. She described an optic neuritis event of her left eyes. She was told at that time that she could have one of a variety of medical conditions, including multiple sclerosis. She was given prednisone by a general practitioner, and her optic neuritis syndrome appeared to have resolved. She remained symptom-free for approximately ten years.

At age 38, she described the recurrence of optic neuritis. She was more descriptive of that event where the vision in the center of her eye appeared distorted.

Peripheral vision was apparently unaffected. Initially, she pursued the care of an ophthalmologist who felt the etiology was once again related possibly to multiple sclerosis, She went on to pursue further evaluation of an internist who later referred her to a neurologist. At the time, she was described as having relapsing-remitting multiple sclerosis. She received a course of ACTH therapy. Initially, the symptoms had resolved, but she went on to describe exacerbations of ophthalmological symptoms between 1982 and 1996, alternating between the right and left eyes. In 1986, she described the onset of "falls." She recalls being affected by the dragging of her left foot on occasion and "stubbing her toe frequently" on the ground. Her right lower extremity was notably stronger than the left. She continued to have a gradual decline from that point on and had no further symptom-free periods.

Since around 1986, she has received the following course of treatments and therapies:
Prednisone (worked initially),
Methylprednisolone (marginal effects),
ACTH (worked initially),
Imuran (no effect),
Cytoxan (uncertain of effect as course of treatment was discontinued due to adverse events),
Betaseron (no effect),
Cladribine (completed in January of 1998, and felt to have had no effect),
Non-allergenic diet (avoidance of dairy products and other foods - no effect),
Bee venom therapy (felt to have increased the strength in legs, through this subsided after 3-4 months)

Currently, she is diagnosed with chronic-progressive multiple sclerosis. She is afflicted with visual impairments, chronic fatigue, difficulty with speech articulation, tremors in the right and left hands, decreased fine motor movement in the right greater than left hands and decreased motor strength in her lower extremities. She is without weight bearing and is confined to a wheelchair. When asked what her major barriers are as far as functional abilities on a day to day basis, she places fatigue at the top of her impairment list followed by speech difficulties and decreased fine motor movement of her hands. She experienced marked spasticity of her lower extremities with the progression of her neurological condition over the course of several years and eventually resorted to a baclofen pump which was implanted in 1997. She has had continuous infusions into her spinal fluid since this time, which has reduced the spasticity considerably in her lower extremities. She receives 85 micrograms of baclofen per day. While the baclofen has ameliorated the spasticity, it is felt that this treatment has lessened her ability to bear weight on her lower extremities.

### Citicoline Response

April 5, 1998 - The patient started on citicoline. She began her treatment at 500 mg per day every morning for the first two weeks. She reported no adverse side effects during this time as well as no notable therapeutic effects. She and her husband maintained a diary to record medication, potential adverse event and neurological changes.

April 22, 1998 - Dosage was increased to 1000 mg per day (500 mg in the a.m. and 500 mg in the p.m.). On the first two days of increased dosage, the patient noted that her balance had been poor and that her fatigue had increased in the early hours of the morning (not her usual time for worsening of fatigue during the day). After two days, however, the patient experienced increased endurance to stay up later into the evenings (normal bedtime was previously 10 p.m.; since the increased dose on citicoline, the patient found herself retiring between midnight and 1:00 a.m.). She was without those early morning fatigue episodes noted on the first few days of therapy, and, in general, was feeling relatively well.

Previously, she was discouraged from using the telephone in that her speech articulation had been poor and perhaps her motivation use the phone had lessened. However, during this period of time, she also found herself both willing and able to conduct telephone inquiries such as to conduct banking activities. She described having a complicated discrepancy with the bank and felt both motivated and capable of articulating the situation to the bank successfully.

May 11, 1998 - Dosage was increased to 2000 mg per day (1000 mg in the a.m., 1000 mg in p.m.) Once again, the patient noticed worsening of fatigue in the morning hours for approximately two days, then returned back to her baseline state of fatigue (generally less fatigue in the morning and a gradual increase in fatigue as the day processed). Once again, it was notable on citicoline (2000 mg/day) that the patient was able to stay up for longer periods during the day. She made a notation in a personal diary that on May 14th she was making several phone calls and described an overall feeling of wellness.

June 5, 1998 - The patient left for a 4 hour car drive to an out-of-state family function. She recalled an inability to endure long car rides for quite some time, and has avoided talking a car trip of this duration in the past five years due to her subsequent exhaustion following these events. She felt well upon arrival despite the long drive. On June 6th, she forgot to take her citicoline dose throughout the day. She described feeling "so-so and not as well as the day before." She also missed taking her citicoline dose on the morning of June 7th. She detected a marked change in her physical well-being in the sense that she was not as "up" and attributed this to the return drive back to her home. They arrived back at her house at approximately 2:30 p.m. The patient described noticing a pronounced difference in her overall state with mainly increased fatigue. She described her tremors being worse, making eating more difficult and she felt, in general, that she had to be more careful as her motor and visual judgment had been off. She took her evening dosage of citicoline (1000 mg) and on the following morning she felt that she was back to baseline while on the drug.

At the end of June, A.H. took a cruise to Alaska with her family, enduring the flight to and from the west coast. She tolerated the trip and extensive traveling quite well.

Her neurologist has no knowledge that she is currently taking citicoline. Prior to taking this medication, she was last seen by her neurologist on April 21, 1998. She was seen recently on July 7, 1998. The neurologist reported noticing an improvement in the patient's eyes in that the "eye movement had improved." The neurologist also noticed that the strength with hand resistance had improved as well. The patient continues to be on citicoline at a dose of 2000 mg per day.

## Claims

1. Use of citicoline for the preparation of a medicament for treating multiple sclerosis, comprising combining an efficacious amount of citicoline, or a pharmaceutically acceptable salt thereof, with a suitable carrier.

2. The use as in claim 1 wherein said efficacious amount of citicoline ranges from 100 mg to 5.000 mg of citicoline per unit dosage.

3. The use as in claim 1, which further comprises combining an efficacious amount of at least one compound selected from the group consisting of glutamate and glycine antagonists, ACTH, glucocorticoids, antiinflammatory drugs, diphenylhydramine, quinine, myotrophin, IGF-1, BDNF, BFGF, beta-interferon, Betaseron, Copaxone, Baclofen, Riluzole, epitopes of myelin basic proteins, calcium channel blockers, alpha₂-receptor therapeutics, CV-5197, Dopamine receptors, Enadoline, Lazabemide, Milnacipran, Nalmefene, RP-60180, SR-57746A, synaptic uptake blockers, cytokines, hormones, AN-100225, AN-100226, CEP-075, Ciliary neurotrophic factor, Endothelial cell factor, Endothelin inhibitors, FR-139317, Interleukin-1 receptor antagonists, lipocortin, JTP-2942, Macrophage-regulating compounds, Motoneuronetrophic factor NBI-117, Nerve growth factor, Neural stem cells, Neutrophil inhibitory factor, NS-506, NT-3, Posatirelin, Schwann cell promoters, sCR1, Somatomedin-1, free radical scavengers, gangliosides, LIGA4, Monosialoganglioside (GM1), ND-37, Siagoside, Ademetionine sulphate tosilate, Ancrod, Apocuanzine, CPC-111, CPC-211, HSV Vectors, KF-17329, KF-19863, LY-178002, MS-153, Nicorandil, N-3393, N-3398, SUN 4757, TJ-8007, VA-045, and imaging and/or contrast agents or a pharmaceutically acceptable salt thereof.

4. The use as in claim 3 wherein said efficacious amount of citicoline ranges from 100 mg to 5.000 mg of citicoline per unit dosage and said efficacious amount of at least a second therapeutic agent ranges from about 10 mg to about 1.000 mg of said at least a second therapeuctic agent per unit dosage.

5. The use as in claim 3 wherein said second therapeutic agent is glucocorticoid, beta-interferon, copaxone, or mixtures thereof.

6. The use as in claim 5 wherein said glucocorticoid is dexamethasone, prednisone or methylprednisolone, or their pharmaceutically acceptable salts, esters, or phosphates thereof.

## Patentansprüche

1. Verwendung von Citicolin zur Herstellung eines Medikaments zur Behandlung von multipler Sklerose, wobei eine wirksam Menge an Citicolin, oder einem pharmazeutisch akzeptierbaren Salz davon, mit einem geeigneten Träger vereinigt wird.

2. Verwendung wie in Anspruch 1, wobei die wirksame Menge an Citicolin von 100 mg bis 5000 mg Citicolin pro Dosiereinheit reicht.

3. Verwendung wie in Anspruch 1, wobei man des weiteren wirksame Mengen von zumindest einer Verbindung, ausgewählt aus der Gruppe, bestehend aus Glutamatund Glycinantagonisten, ACTH, Glucokortikoiden, entzündungshemmenden Arzneistoffen, Diphenylhydramin, Chinin, Myothrophin, IGF-1, BDNF, BFGF, beta-Interferon, Betaseron, Copaxon, Baclofen, Riluzol, Epitopen von basischen Myelinproteinen, Calciumkanalblockern, Alpha₂-Rezeptorterapeutika, CV-5197, Dopaminrezeptoren, Enadolin, Lazabemid, Milnacipran, Nalmefen, RP-60180, SR-57746A, Synaptische Aufnahmeblocker, Cytokine, Hormonen, AN-100225, AN-100226, CEP-075,Ciliary Neurotrophic Factor, Endothelialer Zellfaktor, Endothelininhibitoren, FR-139317, Interleukin-1-Rezeptorantagonisten, Lipocortin, JPT-2942, macrophagenregulierende Verbindungen, Motoneurontrophischem Faktor NBI-117, Nervenwachstumsfaktor, neutrale Stammzellen, Neutrophil Inhibitory Factor, NS-506, NT-3, Posatirelin, Schwann-Zellpromotoren , sCR1, Somatomedin-1, Fänger für freie Radikale, Gangliosiden, LIGA4, Monosialogangliosid (GM1), ND-37, Siagosid, Ademetionintosilatsulphat, Ancord, Apocuanzin, CPC-111, CPC-211, HSV-Vektoren, KF-17329, KF-19863, LY-178002, MS 153, Nicorandil, N-3393, N3398, SUN 4757, TJ-8007, VA-045 und Abbildungsmitteln und/oder Kontrastmitteln sowie pharmazeutisch akzeptierbaren Salzen davon, zugegeben ist.

4. Verwendung wie in Anspruch 3, wobei die wirksame Menge an Citicolin von 100 mg bis 5000 mg Citicolin pro Dosiiereinheit reicht und die wirksame Menge des zumindest einen, zweiten therapeutischen Agens von etwa 10 mg bis etwa 1000 mg des zumindest einen, zweiten therapeutischen Agens reicht.

5. Verwendung wie in Anspruch 3, wobei das zweite, therapeutische Agens ein Glucokortikoid, Betainterferon, Copaxon oder Mischungen daraus ist.

6. Verwendung wie in Anspruch 5, wobei das Glucokortikoid Dexamethason, Prednison oder Methylprednison oder eines ihrer therapeutisch akzeptablen Salze, Ester oder Phosphate ist.

## Revendications

1. Utilisation de citicoline pour la préparation d'un médicament pour le traitement de la sclérose en plaques, comprenant une combinaison d'une quantité efficace de citicoline, ou d'un sel de celle-ci pharmaceutiquement acceptable, avec un véhicule approprié.

2. Utilisation selon la revendication 1, ladite quantité efficace de citicoline variant de 100 mg à 5000 mg de citicoline par unité de dose.

3. Utilisation selon la revendication 1, qui comprend en plus en combinaison une quantité efficace d'au moins un composé sélectionné dans le groupe composé de : antagonistes du glutamate et antagonistes de la glycine, ACTH, glucocorticoïdes, produits pharmaceutiques anti-inflammatoires, diphénylhydramine, quinine, myotrophine, IGF-1, BDNF, BFGF, interféron beta, Betaseron, Copaxone, Baclofen, Riluzole, épitopes des protéines basiques de la myéline, inhibiteurs de canal calcique, agents thérapeutiques de récepteur alpha₂, CV-5197, récepteurs de la dopamine, Enadoline, Lazabemide, Milnacipran, Nalmefene, RP-60180, SR-57746A, inhibiteurs de la recapture synaptique, cytokines, hormones, AN-100225, AN-100226, CEP-075, facteur neurotrophique ciliaire, facteur des cellules endothéliales, inhibiteurs de l'endothéline, FR-139317, antagonistes du récepteur de l'interleukine 1, lipocortine, JTP-2942, composés de régulation des macrophages, facteur motoneurotrophique NBI-117, facteur de croissance nerveux, cellules souches neurales, facteur inhibiteur neutrophile, NS-506, NT-3, Posatireline, inducteurs des cellules de Schwann, sCR1, Somatomédine-1, piégeurs de radicaux libres, gangliosides, LIGA4, monosialoganglioside (GM1), ND-37, Siagoside, tosilate de sulphate d'adémétionine, Ancrod, Apocuanzine, CPC-111, CPC-211, vecteurs HSV, KF-17329, KF-19863, LY-178002, MS-153, Nicorandil, N-3393, N-3398, SUN 4757, TJ-8007, VA-045, et agents d'imagerie et/ou de contraste ou un sel pharmaceutiquement acceptable de ceux-ci.

4. Utilisation selon la revendication 3, ladite quantité efficace de citicoline variant de 100 mg à 5000 mg de citicoline par unité de dose et ladite quantité efficace d'au moins un second agent thérapeutique variant d'environ 10 mg à environ 1000 mg dudit, au moins un, second agent thérapeutique par unité de dose.

5. Utilisation selon la revendication 3, ledit second agent thérapeutique étant un glucocorticoïde, l'interféron beta, la copaxone, ou des mélanges de ceux-ci.

6. Utilisation selon la revendication 5, ledit glucocorticoïde étant la dexaméthasone, la prednisone ou la méthylprednisolone, ou leurs sels pharmaceutiquement acceptables, des esters ou des phosphates de ceux-ci.
